# EUROPEAN PATENT SPECIFICATION

(11) **EP 3 040 023 B1**
(45) Date of publication and mention of the grant of the patent: **26.06.2019**
(21) Application number: 14200629.5
(22) Date of filing: 30.12.2014
(51) Int. Cl.: A61B 5/12, H04R 25/00, A61B 5/00

(54) **Filter device and method of manufacturing a filter device**
Filtervorrichtung und Verfahren zum Herstellen einer Filtervorrichtung
Dispositif de filtrage et procédé de fabrication d'un dispositif de filtre

(43) Date of publication of application: 06.07.2016
(73) Proprietor: Natus Medical Incorporated, Pleasanton, CA 94566 (US)
(72) Inventor: Geertsen, Thomas, DK-4200 Slagelse (DK)
(74) Representative: Zacco Denmark A/S

(56) References cited:
- US-A1- 2006 147 071
- US-A1- 2012 014 548

## Description

### FILTER DEVICE AND METHOD OF MANUFACTURING A FILTER DEVICE

The present disclosure relates to a filter device, e.g. for audiologic test equipment, for preventing cerumen, commonly known as earwax, from entering into a compartment comprising delicate equipment, such as a microphone and/or a speaker. The compartment may be a compartment of a hearing test probe used to perform audiologic tests, such as tympanometric and/or otoacoustic emission tests.

### BACKGROUND

In order to perform an audiologic test a probe needs to be inserted into the ear canal of a person to be tested. The probe may hold audio devices, such as a microphone and/or one or more speakers. These components may be damaged or altered by cerumen from the person's ear. Therefore a filter device may be applied to the probe between the components of the probe and the distal end of the probe which is to be inserted into the ear canal.

The filter device needs to enable sound and air to pass through in order for the audiologic tests to be performed. Concurrently the filter needs to stop cerumen from passing into the probe and thereby potentially damaging components of the probe. Conventional filter devices trap cerumen from the user's ear in order to prevent the cerumen from entering into the probe compartment. However, when cerumen is trapped in the filter, the acoustic properties of the probe may be altered, thereby leading to reduced functionality of the probe.

In a filter device, openings of very small dimensions are desirable. However, it is difficult to carve or pierce a hole in a surface of a desirable small dimension, leading to a high manufacturing cost of the filter device.

A filter device with openings of very small dimensions is disclosed in US 2012/0014548 A1.

### SUMMARY

Despite the known solutions there is still a need for a filter device which effectively prevents cerumen from entering into the probe compartment, and a filter device which is easy and inexpensive to manufacture.

Accordingly, a filter device is provided, as defined in claim 1. The filter device extends along a center axis in a first direction from a first end to a second end. The filter device comprises: a first part having a first filter structure; a second part having a second filter structure; and a first channel extending along the center axis. The first part and the second part are joined together, and the first filter structure and the second filter structure define a filter element with a first set of filter openings for the first channel.

Also disclosed is a method for providing and/or manufacturing a filter device, such as a filter device with a first channel. The method comprises: obtaining a first part with a first filter structure; obtaining a second part with a second filter structure; and joining the first part and the second part. Joining the first part and the second part comprises at least partly inserting the first filter structure into the second filter structure to form a filter element with a first set of filter openings for the first channel.

Further disclosed is a first part and/or a second part for a filter device. Further, a hearing test probe comprising a filter device as disclosed herein is provided.

It is an advantage of the present disclosure that a filter device which may be manufactured cheaply by simple processes such as plastic molding is provided. A filter device which lowers manufacturing costs may provide a filter device which may be exchanged after each use and/or after a few uses, and thereby provides for easy usage and highly reliable and consistent measurements.

### BRIEF DESCRIPTION OF THE DRAWINGS

The above and other features and advantages of the present invention will become readily apparent to those skilled in the art by the following detailed description of exemplary embodiments thereof with reference to the attached drawings, in which:
- Fig. 1: illustrates an exemplary filter device,
- Fig. 2: illustrates a first part and a second part of an exemplary filter device,
- Fig. 3: illustrates a cross section of an exemplary filter device,
- Fig. 4a: is a side view of an exemplary filter device,
- Fig. 4b: is a side view of a first part of an exemplary filter device,
- Fig. 4c: is a side view of a second part of an exemplary filter device,
- Fig. 5a: is an end view of an exemplary filter device,
- Fig. 5b: is an end view of a first part of an exemplary filter device,
- Fig. 5c: is an end view of a second part of an exemplary filter device,
- Fig. 6a: is an end view of an exemplary filter device,
- Fig. 6b: is an end view of a first part of an exemplary filter device,
- Fig. 6c: is an end view of a second part of an exemplary filter device,
- Fig. 7: illustrates an exemplary filter device with a coupling part, and
- Fig. 8: is a flow diagram of an exemplary method of manufacturing a filter device.

### DETAILED DESCRIPTION

The filter device comprises a first channel extending along the center axis. The filter element has a first set of filter openings for the first channel. Additionally or alternatively, the filter device may comprise a second channel extending along the center axis. The filter element may have a second set of filter openings for the second channel. The first set of filter openings may provide fluid communication between the first channel and the surroundings. The second set of filter openings may provide fluid communication between the second channel and the surroundings.

The filter device may comprise a plurality of channels including the first channel, the second channel and/or a third channel extending along the center axis. The channel(s) may at least partly be formed by the first part and/or the second part.

The filter device may have dimensions such that the filter device or part of the filter device may be inserted into a human ear. The filter device may have a length along the center axis, e.g a length less than 15 cm, such as between 0.5 cm and 10 cm, such as between 1 cm and 5 cm, such between 1.5 cm and 3 cm.

The filter element may have a plurality of sets of filter openings for the plurality of channels. The plurality of sets of filter openings for the plurality of channels may include the first set of filter openings for the first channel and the second set of filter openings for the second channel. The filter element may include a third set of filter openings for a third channel. The filter element may include a fourth set of filter openings for a fourh channel. A set of filter openings may comprise one, two, three, four, five six, seven or more filter openings, e.g. in the range from one to ten filter openings.

A filter opening, such as a filter opening of a set of filter openings, may have a cross-sectional shape in a plane perpendicular to the center axis. The cross-sectional shape may be a slit, such as a slit resembling the perimeter or part of a perimeter of a geometrical shape, such as a circle, a rectangle, or an oval. The slit may have a width less than 0.5 mm, such as less than 0.35 mm. such as less than 0.2 mm, such as less than 0.15 mm, such as less than 0.1 mm. The slit may have a constant width and/or a varying width.

The filter device, e.g. the first part, may comprise a tube part with a cavity. The tube part may form outer wall part of one or more channels. The tube part may at least partly enclose the channel(s). The tube part may extend along the center axis. The tube part may have a length less than 10 cm, such as between 0.1 cm and 7 cm, such as between 0.5 cm and 5 cm, such between 1 cm and 3 cm. The tube part may have a circular cross section and/or the tube part may have an oval cross section, and/or the tube part may have a polygonal cross section, and/or the tube part may have a rectangular cross section. The tube part may have a cross-sectional dimension, e.g. a cross-sectional dimension in a plane with a normal parallel to the center axis, such as a diameter and/or a width. The cross-sectional dimension may be selected such that the tube part may be inserted into a human ear canal. The cross-sectional dimension may be less than 2 cm, such as between 0.1 cm and 1.5 cm, such as between 0.2 cm and 1 cm, such as between 0.3 cm and 0.8 cm, such as 0.5 cm.

The filter device, e.g. the second part, may comprise a channel separator extending inside the cavity along the center axis to form the plurality of channels, such as the first channel and the second channel. The channel separator may have a length substantially similar to the length of the tube part. The channel separator may have a length shorter than the length of the tube part, such as 0.1 cm shorter than the length of the tube part. The channel separator may have a length less than 10 cm, such as between 0.1 cm and 7 cm, such as between 0.5 cm and 5 cm, such between 1 cm and 3 cm.

The channel separator may form one or more inner walls, including a first inner wall and/or a second inner wall. The one or more inner walls may radially extend from an intersection, e.g. an intersection extending along the center axis. The channel separator may form four inner walls radially extending from an intersection of the four inner walls extending along the center axis.

One or more inner walls of the channel seperator, such as the first inner wall and/or the second inner wall, may have a width in a direction perpendicular to the center axis. The width may be selected such that the one or more inner walls radially extend to an inner side of the cavity of the tube part. The width of one or more inner walls may be less than 10 mm, such as between 1 mm and 8 mm, such as between 2 mm and 6 mm, such as 3 mm.

The first filter structure may comprise a protruding member and/or a plurality of protruding members, such as a first primary protruding member, a first secondary protruding member, a second primary protruding member, and/or a second secondary protruding member. The protruding member(s) may extend along the center axis.

The protruding member and/or the plurality of protruding members, such as the first primary protruding member, the first secondary protruding member, the second primary protruding member, and/or the second secondary protruding member, may have a distal end.

The protruding member and/or the plurality of protruding members, such as the first primary protruding member, the first secondary protruding member, the second primary protruding member, and/or the second secondary protruding member, may have a length parallel to the center axis between 0.1 and 10 mm, such as between 0.5 mm and 8 mm, such as 1 mm 3 mm or 5 mm.

The second filter structure may comprise an opening and/or a plurality of openings, such as first opening(s) and second opening(s), the first opening(s) e.g. including a first primary opening and/or a first secondary opening, and the second opening(s) e.g. including a second primary opening and/or a second secondary opening. The second filter structure may comprise third opening(s) and fourth opening(s), the third opening(s) e.g. including a third primary opening and/or a third secondary opening, and the fourth opening(s) e.g. including a fourth primary opening and/or a fourth secondary opening.

A protruding member may extend at least partly inside (e.g. overlap of at least 0.5 mm along the center axis) or through an opening of the second filter structure along the center axis to form a filter opening of a set of filter openings. For example, the first primary protruding member may extend at least partly inside or through the first primary opening along the center axis, e.g. with an overlap of at least 0.5 mm, to form a primary first filter opening of the first set of filter openings. Alternatively or additionally, the first secondary protruding member may extend at least partly inside or through the first secondary opening along the center axis, e.g. with an overlap of at least 0.5 mm) to form a secondary first filter opening of the first set of filter openings. Alternatively or additionally, the second primary protruding member may extend inside or through the second primary opening along the center axis to form a primary second filter opening of the second set of filter openings. Alternatively or additionally, the second secondary protruding member may extend inside or through the second secondary opening along the center axis to form a secondary second filter opening of the second set of filter openings. Third protruding member(s) may extend at least partly inside or through third opening(s) of the second filter element along the center axis to form third filter opening(s) of the third set of filter openings. Fourth protruding member(s) may extend at least partly inside or through fourth opening(s) of the second filter element along the center axis to form fourth filter opening(s) of the fourth set of filter openings. A plurality of protruding members may extend into or share the same filter opening.

A protruding member and/or a plurality of protruding members, such as the first primary protruding member, the first secondary protruding member, the second primary protruding member, and/or the second secondary protruding member, may have a circular, oval, oblong, triangular, and/or rectangular cross-section. One or more protruding members with an oblong cross-section may be preferred due to size requirements and acoustic properties.

The cross sectional area of a protruding member and/or the plurality of protruding members may decrease towards the distal end of the protruding member. For example, the cross-sectional area of the first primary protruding member may decrease towards the distal end of the first primary protruding member. This may facilitate moulding of the filter structure and/or simplify moulding tools.

The cross-section of the protruding member and/or the plurality of protruding members may be in a plane having a normal parallel to the center axis.

The opening and/or the plurality of openings, such as the first primary opening, the first secondary opening, the second primary opening, and/or the second secondary opening, may have a circular, oval, oblong, triangular, and/or rectangular cross-section. One or more openings with an oblong or oval cross-section may be preferred due to size requirements and acoustic properties.

The cross-section of the opening and/or the plurality of openings may be in a plane having a normal parallel to the center axis.

An opening, such as an opening of the plurality of openings, such as the first primary opening, the first secondary opening, the second primary opening, and/or the second secondary opening, may have a cross-sectional area. The cross-sectional area of an opening may be selected to allow one or more protruding members to extend inside the opening. For example, the cross-sectional area of an opening may be less than 30 mm², such as between 1 mm² and 20 mm², such as between 2 mm² and 15 mm².

The protruding member and/or the plurality of protruding members, such as the first primary protruding member, the first secondary protruding member, the second primary protruding member, and/or the second secondary protruding member, may comprise one or more support points, such as a first support point and/or a second support point. The one or more support points may when assembled contact an inner sidewall of the opening in the second filter structure. For example, the first support point of the first primary protruding member may contact a first inner sidewall of the first primary opening in the second filter structure when the filter device is assembled.

Providing support points may provide enhanced precision of the filter opening size, reducing variation in acoustic properties. Furthermore, support points may allow for subsequent fusing of the first part and the second part, e.g. by ultrasonic welding.

The second part may comprise a coupling part and/or a plurality of coupling parts, such as a first coupling part and/or a second coupling part, for coupling a channel, such as the first channel and/or the second channel, to a probe channel. The coupling part and/or the plurality of coupling parts may comprise a coupling ridge extending in the first direction and enclosing the opening and/or the plurality of openings, such as the first primary opening, the first secondary opening, the second primary opening, and/or the second secondary opening. For example, the first coupling part may comprise a coupling ridge extending in the first direction and enclosing the first primary opening and/or the first secondary opening. Alternatively and/or additionally, the second coupling part may comprise a coupling ridge extending in the first direction and enclosing the second primary opening and/or the second secondary opening.

The first part and/or the second part may be at least partly coated with a hydrophobic substance. For example, the first filter structure and/or the second filter structure may be at least partly coated with a hydrophobic substance. For example, inside surfaces of the filter openings may be coated with a hydrophobic substance, e.g. the protruding member and/or the plurality of protruding members and/or the opening and/or the plurality of openings may be at least partly coated with a hydrophobic substance.

Coating a surface with a hydrophobic substance will provide cerumen to be attracted to the surface, thus at least partly trapping the cerumen inside the filter openings, preventing or at least reducing the risk of cerumen from moving through and out of the filter openings.

The first part and/or the second part may be at least partly coated with a hydrophilic substance. For example, the first filter structure and/or the second filter structure may be at least partly coated with a hydrophilic substance. For example, the second end may be coated with a hydrophilic substance, e.g. the protruding member and/or the plurality of protruding members and/or the opening and/or the plurality of openings may be at least partly coated with a hydrophilic substance. Coating a surface with a hydrophilic substance will provide cerumen to be repelled from the surface, thus at least partly preventing cerumen from moving into the filter openings.

The length and width of the filter openings may be varied by varying the layout of the first and second filter structures. For example, the sizes of the protruding member and/or plurality of protruding members of the first filter structure, and/or the sizes of the opening and/or plurality of openings of the second filter structure.

For example, the length of the filter openings may be increased, e.g. by increasing the length of the protruding member and/or plurality of protruding members and/or by increasing the length of the opening and/or plurality of openings, thereby enhancing the prevention of cerumen from advancing through the filter openings.

Alternatively or additionally, the width of the filter openings may be decreased, e.g. by increasing the width of the protruding member and/or plurality of protruding members and/or by decreasing the width of the opening and/or plurality of openings, thereby enhancing the prevention of cerumen from moving into the filter openings.

The width and length of the filter openings may alternatively or additionally be varied to achieve desirable acoustic properties of the filter device.

The first part and/or the second part may be made from plastic, such as acrylonitrile butadiene styrene (ABS), a polycarbonate and acrylonitrile butadiene styrene blend (PC-ABS), and/or polyoxymethylene (POM). Obtaining the first part with the first filter structure and/or the second part with the second filter structure may comprise plastic molding of the first part and/or second part, respectively.

Joining the first part and the second part may comprise ultrasonic welding of the first part and the second part. The first part and/or the second part may comprise energy directors for ultrasonic welding of the first part and the second part. An energy director may be a small protrusion in a surface, such as a cone shaped protrusion.

The filter device may be a first filter device and/or a second filter device of a system of filter devices. The system of filter devices may comprise a plurality of filter devices, e.g. including the first filter device and the second filter device. An intermediate space may be provided between the first filter device and the second filter device. The intermediate space may be provided to trap cerumen between the first filter device and the second filter device.

The figures are schematic and simplified for clarity, and merely show details which are essential to the understanding of the invention, while other details have been left out. Throughout, the same reference numerals are used for identical or corresponding parts.

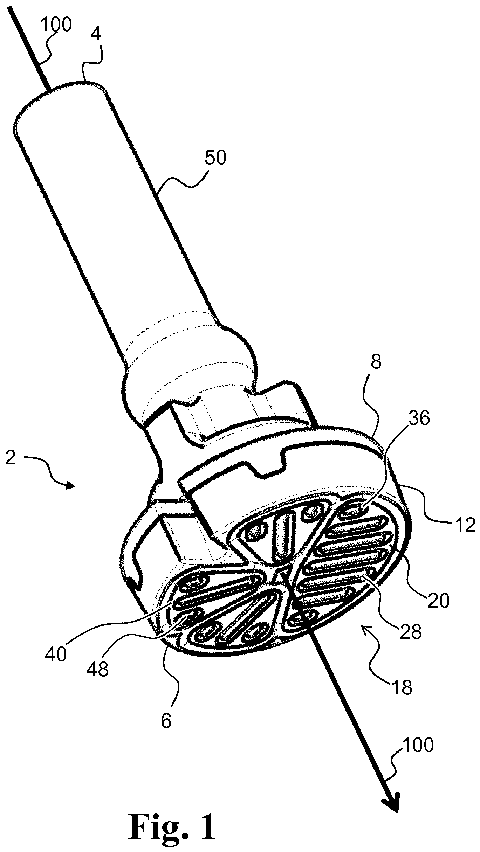

Fig. 1 illustrates an exemplary filter device 2 for a hearing test probe (not shown). The filter device 2 extends along a center axis 100 in a first direction from a first end 4 to a second end 6. The filter device 2 comprises a first part 8 and a second part 12. The filter device comprises a first channel extending along the center axis 100. The first part 8 comprises a tube part 50, wherein the first channel is extending.

The first part 8 has a first filter structure, and the second 12 has a second filter structure. The first part 8 and the second part 12 are joined together, and the first filter structure and the second filter structure define a filter element 18 with a first set of filter openings 20 for a first channel. The first set of filter openings 20 comprises a plurality of first filter openings. The first set of filter openings 20 comprises a primary first filter opening 28 and a secondary first filter opening 36.

The filter element 18 further comprises a second set of filter openings 40 for a second channel. The second set of filter openings 40 comprises a plurality of second filter openings. The second set of filter openings 40 comprises a primary second filter opening 48. The second channel is extending inside the tube part 50.

The filter element 18 may comprise a plurality of sets of filter openings for a plurality of channels. The filter element 18 as illustrated comprises four sets of filter openings for four channels. A plurality of sets of filter openings for a plurality of channels may provide sets of filter openings and channels for different purposes. For example, the first set of filter openings 20 may be intended for outcoming sound, e.g. connected to a speaker, and the second set of filter openings 40 may be intended for incoming sound, e.g. connected to a microphone, and a third set of filter openings may be intended for pressurizing, e.g. connected to a pump.

In an exemplary filter device (not shown), the second part 12 may comprise one or more coupling parts for coupling a channel to a probe channel, e.g. the second part 12 may comprise a first coupling part for coupling the first channel to a probe channel and/or the second part 12 may comprise a second coupling part for coupling the second channel to a probe channel. The first coupling part may comprise a coupling ridge extending in the first direction and enclosing the primary first filter opening 28 and/or the secondary first filter opening 36, such as enclosing the first set of filter openings 20. The second coupling part may comprise a coupling ridge extending in the first direction and enclosing the primary second filter opening 48, such as enclosing the second set of filter openings 40.

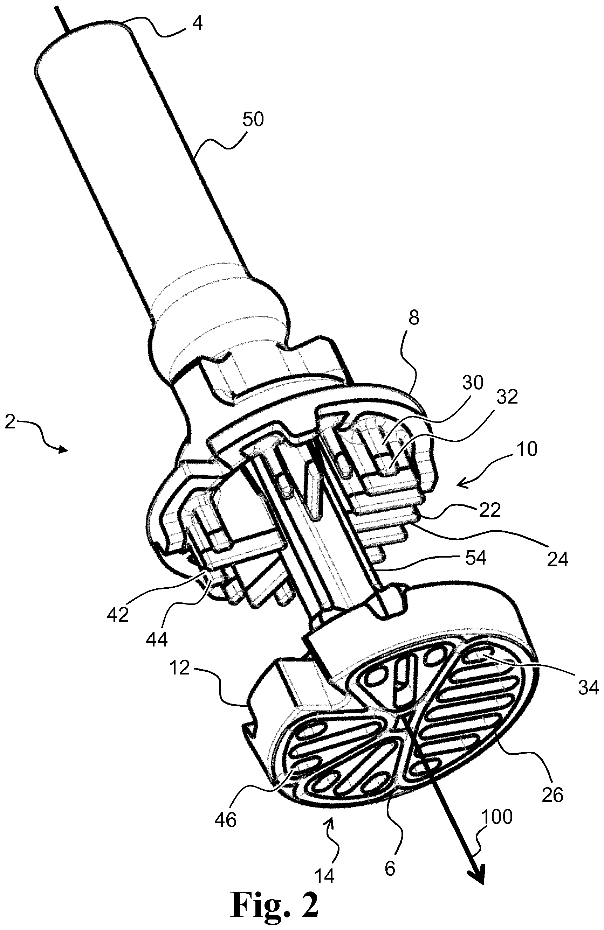

Fig. 2 illustrates an exemplary filter device 2 prior to joining the first part 8 and the second part 12 to form the filter device 2 as illustrated in Fig. 1. The filter device 2 comprises the first part 8 having a first filter structure 10 and the second part 12 having a second filter structure 14.

The first part 8 comprises a tube part 50 with a cavity, and the second part 12 comprises a channel separator 54 with four inner walls extending inside the cavity along the center axis 100. The inner walls of the channel separator 54 extending inside the cavity form four channels including a first channel and a second channel.

The first filter structure 10 comprises a first primary protruding member 22 having a distal end 24. The second filter structure comprises a first primary opening 26. After joining the first part 8 and the second part 12, the first primary protruding member 22 extends at least partly inside or through the first primary opening 26 along the center axis to form a primary first filter opening of the first set of filter openings for the first channel as illustrated in Fig. 1.

The first filter structure 10 comprises a plurality of protruding members, and the second filter structure 14 comprises a plurality of openings.

The first filter structure 10 comprises a first secondary protruding member 30 having a distal end 32. The second filter structure comprises a first secondary opening 34. After joining the first part 8 and the second part 12, the first secondary protruding member 30 extends at least partly inside or through the first secondary opening 34 along the center axis to form a secondary first filter opening of the first set of filter openings for the first channel as illustrated in Fig. 1.

The first filter structure 10 comprises a second primary protruding member 42 having a distal end 44. The second filter structure comprises a second primary opening 46. After joining the first part 8 and the second part 12, the second primary protruding member 42 extends at least partly inside or through the second primary opening 46 along the center axis to form a primary second filter opening of the second set of filter openings for the second channel as illustrated in Fig. 1.

After joining the first part 8 and the second part 12, the protruding members of the first filter structure 10, such as the first primary protruding member 22, the first secondary protruding member 30, and/or the second primary protruding member 42 are configured to overlap along the center axis with their respective openings of the second filter structure, such as the first primary opening 26, the first secondary opening 34, and/or the second primary opening 46.

In an exemplary filter device (not shown), the second part 12 may comprise one or more coupling parts for coupling a channel to a probe channel, e.g. the second part 12 may comprise a first coupling part for coupling the first channel to a probe channel and/or the second part 12 may comprise a second coupling part for coupling the second channel to a probe channel. The first coupling part may comprise a coupling ridge extending in the first direction and enclosing the first primary opening 26 and/or the first secondary opening 34. The second coupling part may comprise a coupling ridge extending in the first direction and enclosing the second primary opening 46.

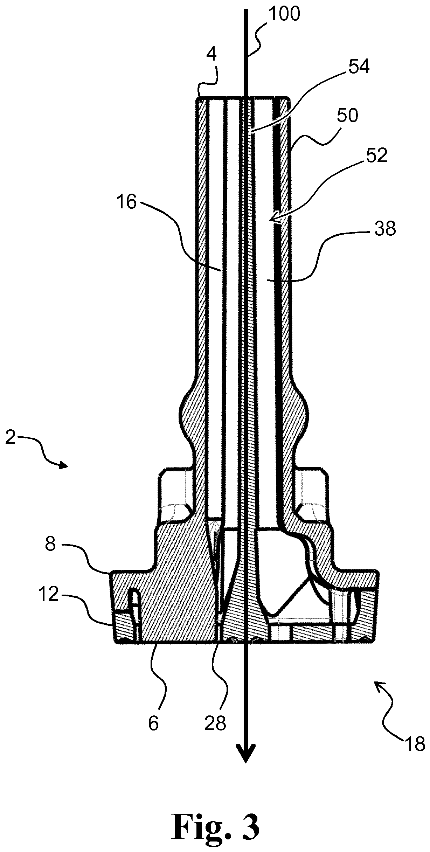

Fig. 3 illustrates a cross section of an exemplary filter device 2. The filter device 2 comprises a first channel 16 and a second channel 38. Fig. 3 furthermore illustrates the first part 8 comprising a tube part 50 having a cavity 52, and the second part 12 comprising a channel separator 54 extending inside the cavity 52 along the center axis 100. The channel separator 54 extending inside the cavity 52 forms the first channel 16 and the second channel 38. The first part 8 and the second part 12 are joined together to form a filter element 18 comprising a plurality of filter openings. The filter element 18 comprises a primary first filter opening 28 for the first channel 16. The primary first filter opening 28 may be part of a first set of filter openings for the first channel 16 as described in relation to Fig. 1.

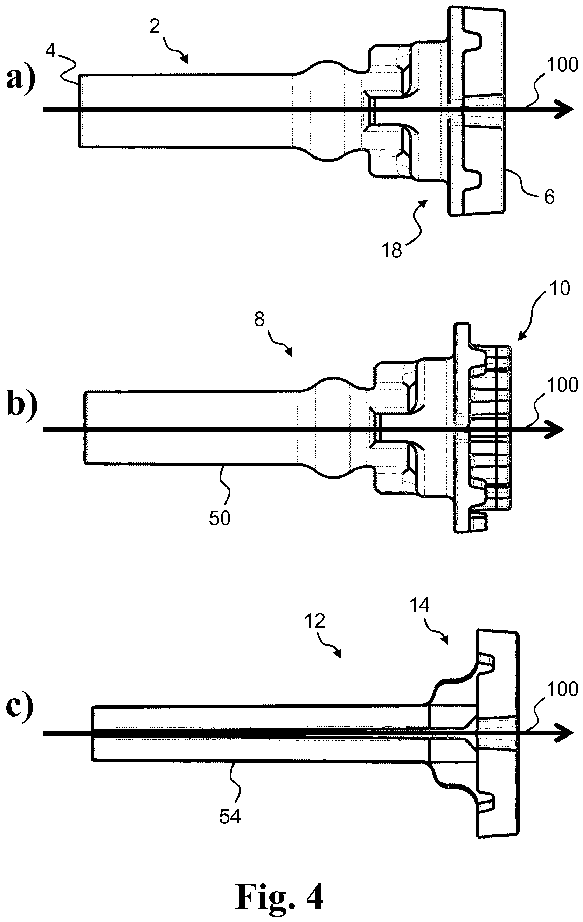

Figs. 4a-4c are side views of an exemplary filter device 2, an exemplary first part 8, and an exemplary second part 12, respectively. Fig. 4a illustrates the filter device 2. Fig. 4b illustrates the first part 8 of the filter device 2. The first part 8 having a first filter structure 10. Fig. 4c illustrates the second part 12 of the filter device 2. The second part having a second filter structure 14. Fig. 4a illustrates the filter device 2 wherein the first part 8 and the second part 12 are joined together. Thereby, the first filter structure 10 and the second filter 14 structure define a filter element 18 of the filter device 2.

Also illustrated in Figs. 4a-4c are that the first part 8 comprises a tube part 50, and the second part 12 comprises a channel separator 54. After joining the first part 8 and the second part 12, the channel separator 54 extends inside a cavity of the tube part 50 along the center axis 100, forming a first channel and a second channel. Optionally, the channel separator 54 may comprise a plurality of separator walls to form a plurality of channels, e.g. the channel separator 54 may comprise four separator walls to form a first channel, a second channel, a third channel, and a fourth channel.

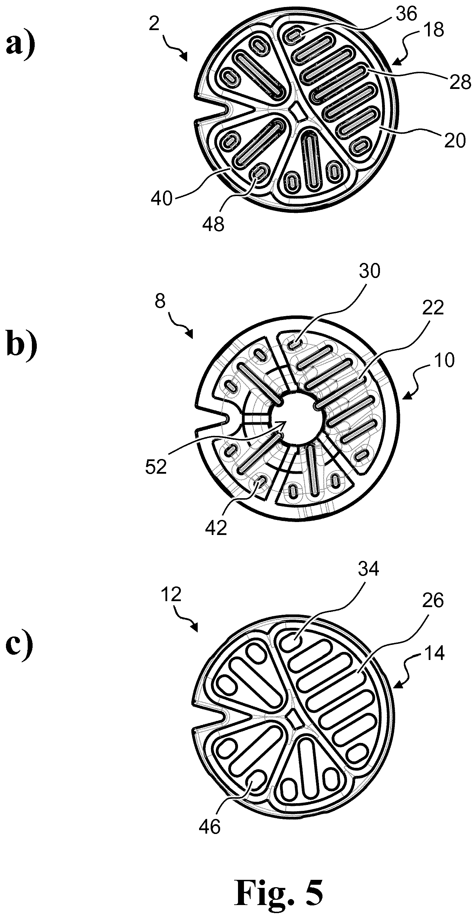

Figs. 5a-5c illustrates an exemplary filter device 2, an exemplary first part 8, and an exemplary second part 12, respectively, shown from the second end 6 (Fig. 1).

Fig. 5a illustrates the filter device 2, wherein the filter element 18 comprises a first set of filter openings 20 for a first channel and a second set of filter openings 40 for a second channel. The first set of filter openings 20 comprises a primary first filter opening 28 and a secondary first filter opening 36. The second set of filter openings 40 comprises a primary second filter opening 48.

Fig. 5b illustrates the first part 8 of the filter device 2. The first part 8 having a first filter structure 10. The first filter structure 10 comprises a plurality of protruding members, including a first primary protruding member 22, a first secondary protruding member 30, and a second primary protruding member 42. Also shown is the cavity 52 of a tube part of the first part 8. As shown in Fig. 5b, a protruding member, such as the first primary protruding member 22, the first secondary protruding member 30, and/or the second primary protruding member 42, may have an oval or oblong cross-section. Alternatively, a protruding member, such as the first primary protruding member 22, the first secondary protruding member 30, and/or the second primary protruding member 42, may be circular, triangular and/or rectangular cross-section.

Fig. 5c illustrates the second part 12 of the filter device 2. The second part having a second filter structure 14. The second filter structure 14 comprises a plurality of openings, including a first primary opening 26, a first secondary opening 34, and a second primary opening 46.

The openings of the second filter structure 14, such as the first primary opening 26, the first secondary opening 34, and/or the second primary opening 46 are slightly bigger than their respective protruding member of the first filter structure 10, such as the first primary protruding member 22, the first secondary protruding member 30, and/or the second primary protruding member 42, e.g. an opening 26, 34, 46 has a cross-sectional area slightly bigger than its respective protruding member 22, 30, 42.

In some exemplary filter devices (not shown) a protruding member of the first filter structure 10, such as the first primary protruding member 22, the first secondary protruding member 30, and/or the second primary protruding member 42, may comprise one or more support points including a first support point. After joining the first part 8 and the second part 12, the first support point may contact a first inner sidewall of an opening of the second filter structure 14, such as the first primary opening 26, the first secondary opening 34, and/or the second primary opening 46.

After joining the first part 8 and the second part 12 the first filter structure 10 and the second filter structure 12 defines the filter element 18, as shown in Fig. 5a. After joining the first part 8 and the second part 12, the first primary protruding member 22 extends at least partly inside or through the first primary opening 26 to form the primary first filter opening 28. After joining the first part 8 and the second part 12, the first secondary protruding member 30 extends at least partly inside or through the first secondary opening 34 to form the secondary first filter opening 36. After joining the first part 8 and the second part 12, the second primary protruding member 42 extends at least partly inside or through the second primary opening 46 to form the primary second filter opening 48.

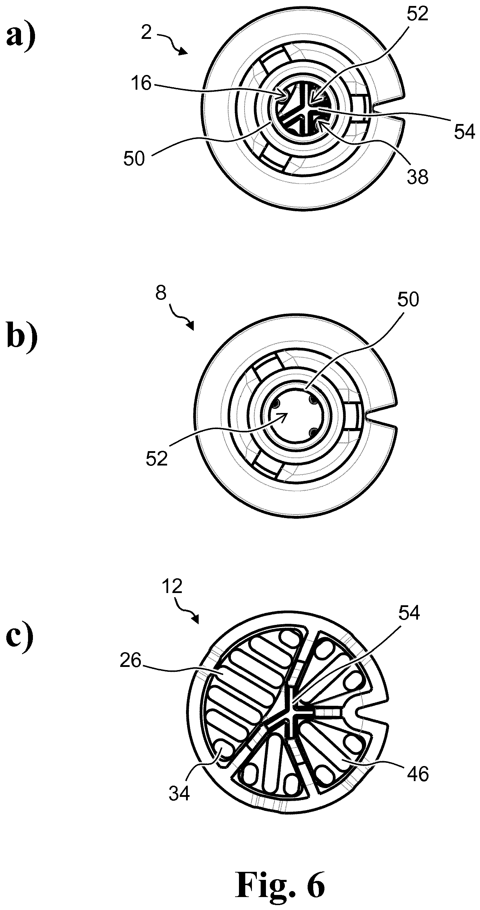

Figs. 6a-6c illustrates an exemplary filter device 2, an exemplary first part 8, and an exemplary second part 12, respectively, shown from the first end 4 (Fig. 1).

Fig. 6a illustrates the filter device 2 comprising a first channel 16 and a second channel 38. Fig. 6b illustrates the first part 8 of the filter device 2. The first part 8 comprises a tube part 50 with a cavity 52. Fig. 6c illustrates the second part 12 of the filter device 2. The second part 12 comprises a channel separator 54. After joining the first part 8 and the second part 12, the filter device 2 comprises a plurality of channels including the first channel 16 and a second channel 38, as shown in Fig. 6a. The first channel 16 and the second channel 38 are separated by the channel separator 54 extending inside the cavity 52 of the tube part 50.

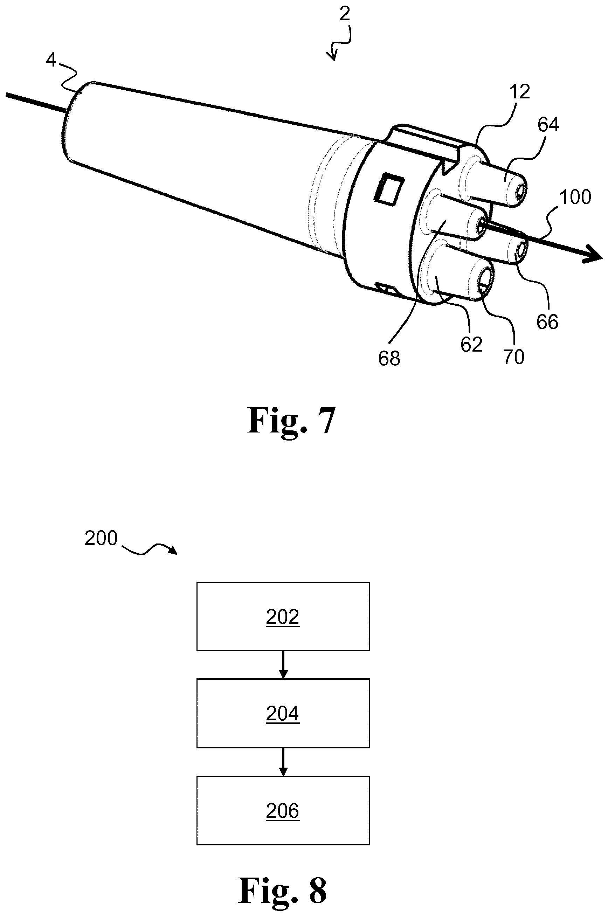

Fig. 7 illustrates an exemplary filter device 2, wherein the second part 12 comprises one or more coupling parts including a first coupling part 62. The second part 12 comprises a plurality of coupling parts including the first coupling part 62, a second coupling part 64, a third coupling part 66 and a fourth coupling part 68, for coupling respective channels and filter openings to respective probes/probe channels. The filter device may comprise a coupling member including the coupling part(s). The coupling member may be a separate member, e.g. made in a material different from the first and second parts. The first coupling part is configured for coupling the first channel to a probe channel and/or for coupling the filter device 2 to a probe. The plurality of coupling parts may be configured for coupling the plurality of channels of the filter device to a plurality of channels of the probe. A coupling part may comprise a coupling ridge extending in the first direction. The first coupling part 62 comprises a first coupling ridge 70 extending in the first direction..

Fig. 8 is a flow diagram of an exemplary method 200 of manufacturing a filter device with a first channel. The method 200 comprises obtaining 202 a first part with a first filter structure, obtaining 204 a second part with a second filter structure, and joining 206 the first part and the second part.

Joining 206 the first part and the second part comprises at least partly inserting the first filter structure into the second filter structure to form a filter element with a first set of filter openings for the first channel.

Obtaining 202 the first part may comprise plastic molding of the first part and/or obtaining 204 the second part may comprise plastic molding of the second part.

Joining 206 the first part and the second part may comprise ultrasonic welding of the first part and the second part. For example, joining 206 the first part and the second part may comprise inserting the first filter structure into the second filter structure, and subsequently ultrasonic welding the first part and the second part.

### LIST OF REFERENCES

- 2: filter device
- 4: first end
- 6: second end
- 8: first part
- 10: first filter structure
- 12: second part
- 14: second filter structure
- 16: first channel
- 18: filter element
- 20: first set of filter openings
- 22: first primary protruding member
- 24: distal end
- 26: first primary opening
- 28: primary first filter opening
- 30: first secondary protruding member
- 32: distal end
- 34: first secondary opening
- 36: secondary first filter opening
- 38: second chanel
- 40: second set of filter openings
- 42: second primary protruding member
- 44: distal end
- 46: second primary opening
- 48: primary second filter opening
- 50: tube part
- 52: cavity
- 54: channel seperator
- 62: first coupling part
- 64: second coupling part
- 66: third coupling part
- 68: fourth coupling part
- 70: coupling ridge
- 100: center axis
- 200: method of manufacturing a filter device
- 202: obtaining a first part
- 204: obtaining a second part
- 206: joining the first part and the second part

## Claims

1. A filter device (2) for a hearing test probe, the filter device (2) extending along a center axis (100) in a first direction from a first end (4) to a second end (6) and comprising:
- a first part (8) having a first filter structure (10),
- a second part (12) having a second filter structure (14),
- a first channel (16) extending along the center axis (100),
wherein the first part (8) and second part (12) are joined together, and the first filter structure (10) and the second filter structure (14) define a filter element (18) with a first set of filter openings (20) for the first channel (16), and wherein the first filter structure (10) comprises a first primary protruding member (22) having a distal end (24), and wherein the second filter structure (14) comprises a first primary opening (26),
the first primary protruding member (22) extending at least partly inside the first primary opening (26) along the center axis (100) to form a primary first filter opening (28) of the first set of filter openings (20),
**characterised in that**
the first filter structure (10) comprises a first secondary protruding member (30), and the second filter structure (14) comprises a first secondary opening (34), the first secondary protruding member (30) extending at least partly inside the first secondary opening (34) along the center axis (100) to form a secondary first filter opening (36) of the first set of filter openings (20).

2. Filter device according to claim 1, wherein the first primary protruding member (22) has a circular, oval, oblong, triangular, or rectangular cross-section.

3. Filter device according to any of claims 1-2, wherein the first primary protruding member (22) comprises one or more support points including a first support point, and wherein the first support point contacts a first inner sidewall of the first primary opening (26) in the second filter structure (14).

4. Filter device according to any of claims 1-3, wherein the second part (12) comprises a first coupling part (62) for coupling the first channel (16) to a probe channel, the first coupling part (62) comprising a coupling ridge (70) extending in the first direction and enclosing the first primary opening (26).

5. Filter device according to any of claims 1-4, wherein the cross-sectional area of the first primary protruding member (22) decreases towards its distal end (24).

6. Filter device according to any of the preceding claims, wherein the filter device comprises a second channel (38) extending along the center axis (100), and wherein the filter element (18) has a second set of filter openings (40) for the second channel (38).

7. Filter device according to claim 6, wherein the first part (8) comprises a tube part (50) with a cavity (52), and the second part (12) comprises a channel separator (54) extending inside the cavity (52) along the center axis (100) to form the first channel (16) and the second channel (38).

8. Filter device according to any of claims 6-7, wherein the first filter structure comprises (10) a second primary protruding member (42), and wherein the second filter structure (14) comprises a second primary opening (46), the second primary protruding member (42) extending inside the second primary opening (46) along the center axis (100) to form a primary second filter opening (48) of the second set of filter openings (40).

9. Filter device according to any of the preceding claims, wherein the first part (8) and/or the second part (12) is at least partly coated with a hydrophobic substance.

10. Hearing test probe comprising a filter device according to any of claims 1-9.

11. Method (200) of manufacturing a filter device with a first channel, the method comprising:
- Obtaining (202) a first part with a first filter structure, wherein the first filter structure comprises a first primary protruding member having a distal end, and the first filter structure comprises a first secondary protruding member;
- Obtaining (204) a second part with a second filter structure, wherein the second filter structure comprises a first primary opening, and the second filter structure comprises a first secondary opening; and
- Joining (206) the first part and the second part, wherein joining the first part and the second part comprises at least partly inserting the first filter structure into the second filter structure to form a filter element with a first set of filter openings for the first channel, wherein the first primary protruding member extending at least partly inside the first primary opening to form a primary first filter opening of the first set of filter openings, and wherein the first secondary protruding member extending at least partly inside the first secondary opening to form a secondary first filter opening of the first set of filter openings.

12. Method according to claim 11, wherein obtaining (202) the first part with the first filter structure comprises plastic molding of the first part.

13. Method according to any of claims 11-12, wherein the joining (206) comprises ultrasonic welding of the first part and the second part.

## Patentansprüche

1. Filtervorrichtung (2) für eine Hörtestsonde, wobei sich die Filtervorrichtung (2) entlang einer Mittelachse (100) in einer ersten Richtung von einem ersten Ende (4) zu einem zweiten Ende (6) erstreckt und Folgendes umfasst:
- einen ersten Teil (8) mit einer ersten Filterstruktur (10),
- einen zweiten Teil (12) mit einer zweiten Filterstruktur (14),
- einen ersten Kanal (16), der sich entlang der Mittelachse (100) erstreckt,
wobei der erste Teil (8) und der zweite Teil (12) miteinander verbunden sind, und die erste Filterstruktur (10) und die zweite Filterstruktur (14) ein Filterelement (18) mit einem ersten Satz von Filteröffnungen (20) für den ersten Kanal (16) definieren, und wobei die erste Filterstruktur (10) ein erstes primäres vorspringendes Element (22) mit einem distalen Ende (24) umfasst, und wobei die zweite Filterstruktur (14) eine erste primäre Öffnung (26) umfasst,
wobei sich das erste primäre vorspringende Element (22) zumindest teilweise in der ersten primären Öffnung (26) entlang der Mittelachse (100) erstreckt, um eine primäre erste Filteröffnung (28) des ersten Satzes von Filteröffnungen (20) zu bilden,
**dadurch gekennzeichnet, dass**
die erste Filterstruktur (10) ein erstes sekundäres vorspringendes Element (30) umfasst, und die zweite Filterstruktur (14) eine erste sekundäre Öffnung (34) umfasst, wobei sich das erste sekundäre vorspringende Element (30) zumindest teilweise in der ersten sekundären Öffnung (34) entlang der Mittelachse (100) erstreckt, um eine sekundäre erste Filteröffnung (36) des ersten Satzes von Filteröffnungen (20) zu bilden.

2. Filtervorrichtung nach Anspruch 1, wobei das erste primäre vorspringende Element (22) einen kreisförmigen, ovalen, länglichen, dreieckigen oder rechteckigen Querschnitt aufweist.

3. Filtervorrichtung nach einem der Ansprüche 1-2, wobei das erste primäre vorspringende Element (22) einen oder mehrere Unterstützungspunkte einschließlich eines ersten Unterstützungspunkts umfasst, und wobei der erste Unterstützungspunkt mit einer ersten inneren Seitenwand der ersten primären Öffnung (26) in der zweiten Filterstruktur (14) in Kontakt kommt.

4. Filtervorrichtung nach einem der Ansprüche 1-3, wobei der zweite Teil (12) einen ersten Kupplungsteil (62) zur Kupplung des ersten Kanals (16) zu einem Sondenkanal umfasst, wobei der erste Kupplungsteil (62) eine Kupplungsrippe (70) umfasst, die sich in der ersten Richtung erstreckt und die erste primäre Öffnung (26) umgibt.

5. Filtervorrichtung nach einem der Ansprüche 1-4, wobei der Querschnittsbereich des ersten primären vorspringenden Elements (22) gegen sein distales Ende (24) abnimmt.

6. Filtervorrichtung nach einem der vorgehenden Ansprüche, wobei die Filtervorrichtung einen zweiten Kanal (38) umfasst, der sich entlang der Mittelachse (100) erstreckt, und wobei das Filterelement (18) einen zweiten Satz von Filteröffnungen (40) für den zweiten Kanal (38) aufweist.

7. Filtervorrichtung nach Anspruch 6, wobei der erste Teil (8) einen Rohrteil (50) mit einem Hohlraum (52) umfasst, und der zweite Teil (12) einen Kanalseparator (54) umfasst, der sich innerhalb des Hohlraums (52) entlang der Mittelachse (100) erstreckt, um den ersten Kanal (16) und den zweiten Kanal (38) zu bilden.

8. Filtervorrichtung nach einem der Ansprüche 6-7, wobei die erste Filterstruktur (10) ein zweites primäres vorspringendes Element (42) umfasst, und wobei die zweite Filterstruktur (14) eine zweite primäre Öffnung (46) umfasst, wobei sich das zweite primäre vorspringende Element (42) in der zweiten primären Öffnung (46) entlang der Mittelachse (100) erstreckt, um eine primäre zweite Filteröffnung (48) des zweiten Satzes von Filteröffnungen (40) zu bilden.

9. Filtervorrichtung nach einem der vorgehenden Ansprüche, wobei der erste Teil (8) und/oder der zweite Teil (12) zumindest teilweise mit einer hydrophoben Substanz beschichtet ist.

10. Hörtestsonde umfassend eine Filtervorrichtung nach einem der Ansprüche 1-9.

11. Verfahren (200) zur Herstellung einer Filtervorrichtung mit einem ersten Kanal, welches Verfahren Folgendes umfasst:
- Erhalten (202) eines ersten Teils mit einer ersten Filterstruktur, wobei die erste Filterstruktur ein erstes primäres vorspringendes Element mit einem distalen Ende umfasst, und die erste Filterstruktur ein erstes sekundäres vorspringendes Element umfasst;
- Erhalten (204) eines zweiten Teils mit einer zweiten Filterstruktur, wobei die zweite Filterstruktur eine erste primäre Öffnung umfasst, und die zweite Filterstruktur eine erste sekundäre Öffnung umfasst; und
- Verbinden (206) des ersten Teils und des zweiten Teils, wobei das Verbinden des ersten Teils und des zweiten Teils zumindest teilweise Einführung der ersten Filterstruktur in die zweite Filterstruktur umfasst, um ein Filterelement mit einem ersten Satz von Filteröffnungen für den ersten Kanal zu bilden, wobei sich das erste primäre vorspringende Element zumindest teilweise in der ersten primären Öffnung erstreckt, um eine primäre erste Filteröffnung des ersten Satzes von Filteröffnungen zu bilden, und wobei sich das erste sekundäre vorspringende Element zumindest teilweise in der ersten sekundären Öffnung erstreckt, um eine erste sekundäre Filteröffnung des ersten Satzes von Filteröffnungen zu bilden.

12. Verfahren nach Anspruch 11, wobei das Erhalten (202) des ersten Teils mit der ersten Filterstruktur Kunststoffformen des ersten Teils umfasst.

13. Verfahren nach einem der Ansprüche 11-12, wobei das Verbinden (206) Ultraschallschweißen des ersten Teils und des zweiten Teils umfasst.

## Revendications

1. Dispositif de filtrage (2) pour une sonde de test auditif, le dispositif de filtrage (2) s'étendant le long d'un axe central (100) dans une première direction d'une première extrémité (4) à une deuxième extrémité (6) et comprenant :
- une première partie (8) comportant une première structure de filtrage (10),
- une deuxième partie (12) comportant une deuxième structure de filtrage (14),
- un premier canal (16) s'étendant le long de l'axe central (100),
dans lequel la première partie (8) et la deuxième partie (12) sont jointes, et la première structure de filtrage (10) et la deuxième structure de filtrage (14) définissent un élément filtrant (18) avec un premier ensemble d'ouvertures de filtrage (20) pour le premier canal (16), dans lequel la première structure de filtrage (10) comprend un premier élément en saillie primaire (22) ayant une extrémité distale (24), et dans lequel la deuxième structure de filtrage (14) comprend une première ouverture primaire (26),
le premier élément en saillie primaire (22) s'étendant au moins partiellement à l'intérieur de la première ouverture primaire (26) le long de l'axe central (100) pour former une première ouverture de filtrage primaire (28) du premier ensemble d'ouvertures de filtrage (20),
**caractérisé en ce que**
la première structure de filtrage (10) comprend un premier élément en saillie secondaire (30), et la deuxième structure de filtrage (14) comprend une première ouverture secondaire (34), le premier élément en saillie secondaire (30) s'étendant au moins partiellement à l'intérieur de la première ouverture secondaire (34) le long de l'axe central (100) pour former une première ouverture de filtrage secondaire (36) du premier ensemble d'ouvertures de filtrage (20).

2. Dispositif de filtrage selon la revendication 1, dans lequel le premier élément en saillie primaire (22) a une section transversale circulaire, ovale, oblongue, triangulaire ou rectangulaire.

3. Dispositif de filtrage selon l'une quelconque des revendications 1 à 2, dans lequel le premier élément en saillie primaire (22) comprend un ou plusieurs points de support, y compris un premier point de support, et dans lequel le premier point de support est en contact avec une première paroi latérale intérieure de la première ouverture primaire (26) dans la deuxième structure de filtrage (14).

4. Dispositif de filtrage selon l'une quelconque des revendications 1 à 3, dans lequel la deuxième partie (12) comprend une première partie de couplage (62) pour coupler le premier canal (16) à un canal de sonde, la première partie de couplage (62) comprenant une arête de couplage (70) s'étendant dans la première direction et enfermant la première ouverture primaire (26).

5. Dispositif de filtrage selon l'une quelconque des revendications 1 à 4, dans lequel l'étendue transversale du premier élément en saillie primaire (22) diminue en direction de son extrémité distale (24).

6. Dispositif de filtrage selon l'une quelconque des revendications précédentes, dans lequel le dispositif de filtrage comprend un deuxième canal (38) s'étendant le long de l'axe central (100), et dans lequel l'élément filtrant (18) a un deuxième ensemble d'ouvertures de filtrage (40) pour le deuxième canal (38).

7. Dispositif de filtrage selon la revendication 6, dans lequel la première partie (8) comprend une partie de tube (50) avec une cavité (52), et la deuxième partie (12) comprend un séparateur de canal (54) s'étendant à l'intérieur de la cavité (52) le long de l'axe central (100) pour former le premier canal (16) et le deuxième canal (38).

8. Dispositif de filtrage selon l'une quelconque des revendications 6 à 7, dans lequel la première structure de filtrage (10) comprend un deuxième élément en saillie primaire (42), et dans lequel la deuxième structure de filtrage (14) comprend une deuxième ouverture primaire (46), le deuxième élément en saillie primaire (42) s'étendant à l'intérieur de la deuxième ouverture primaire (46) le long de l'axe central (100) pour former une deuxième ouverture de filtrage primaire (48) du deuxième ensemble d'ouvertures de filtrage (40).

9. Dispositif de filtrage selon l'une quelconque des revendications précédentes, dans lequel la première partie (8) et / ou la deuxième partie (12) est au moins partiellement revêtue d'une substance hydrophobe.

10. Sonde de test auditif comprenant un dispositif de filtrage selon l'une quelconque des revendications 1 à 9.

11. Procédé (200) de fabrication d'un dispositif de filtrage avec un premier canal, le procédé comprenant :
- l'obtention (202) d'une première partie avec une première structure de filtrage, la première structure de filtrage comprenant un premier élément en saillie primaire ayant une extrémité distale, et la première structure de filtrage comprenant un premier élément en saillie secondaire ;
- l'obtention (204) d'une deuxième partie avec une deuxième structure de filtrage, la deuxième structure de filtrage comprenant une première ouverture primaire, et la deuxième structure de filtrage comprenant une première ouverture secondaire ; et
- la jonction (206) de la première partie et de la deuxième partie, la jonction de la première partie et de la deuxième partie comprenant l'insertion au moins partielle de la première structure de filtrage dans la deuxième structure de filtrage pour former un élément filtrant avec un premier ensemble d'ouvertures de filtrage pour le premier canal, le premier élément en saillie primaire s'étendant au moins partiellement à l'intérieur de la première ouverture primaire pour former une première ouverture de filtrage primaire du premier ensemble d'ouvertures de filtrage, et le premier élément en saillie secondaire s'étendant au moins partiellement à l'intérieur de la première ouverture secondaire pour former une première ouverture de filtrage secondaire du premier ensemble d'ouvertures de filtrage.

12. Procédé selon la revendication 11, dans lequel l'obtention (202) de la première partie avec la première structure de filtrage comprend un moulage en plastique de la première partie.

13. Procédé selon l'une quelconque des revendications 11 à 12, dans lequel la jonction (206) comprend un soudage par ultrasons de la première partie et de la deuxième partie.
